# EUROPEAN PATENT APPLICATION

(11) **EP 4 293 094 A1**
(43) Date of publication of application: **20.12.2023**
(21) Application number: 22179530.5
(22) Date of filing: 17.06.2022
(51) Int. Cl.: C11B 9/00, C07B 37/02

(54) **USE OF LIMONENE DIALDEHYDE MIXTURES AS AROMA INGREDIENT**

(71) Applicant: BASF SE, 67056 Ludwigshafen am Rhein (DE)
(72) Inventor: LIMBURG, Carolin, 67056 Ludwigshafen am Rhein (DE); BRU ROIG, Miriam, 67056 Ludwigshafen am Rhein (DE); PELZER, Ralf, 67056 Ludwigshafen am Rhein (DE); GARLICHS, Florian, 67056 Ludwigshafen am Rhein (DE)
(74) Representative: Reitstötter Kinzebach

(57) **Abstract**

The present invention relates mixture of compound of formula (I), compound of formula (II) and compound of formula (III) and its use as aroma ingredient. The mixture is used to impart an aroma impression which is reminiscent of Agrumic note, Chrysanthemum note, Floral note, Muguet note, Watery note, Waxy note, citric note, Lemon note, Aldehydic note, Green note or a combination of two or more of these note. It is also used for enhancing and/or modifying the aroma of a composition. The present invention is further directed to a composition comprising the mixture and (i) at least one aroma chemical different from compound of formula (I), (II) and (III) or (ii) at least one non-aroma chemical carrier, or (iii) both (i) and (ii).

## Description

### Field of invention

The present invention relates to the use of the mixture of compound of formula (I) and compound of formula (II) and further compound of formula (III) as an aroma ingredient. The mixture is used to impart an aroma impression which is reminiscent of Agrumic note, Chrysanthemum note, Floral note, Muguet note, Watery note, Waxy note, Citric note, Lemon note, Aldehydic note, Green note or a combination of two or more notes. It is also used for enhancing and/or modifying the aroma of a composition. The present invention is further directed to a composition comprising the mixture and (i) at least one aroma chemical different from compound of formula (I), (II) and (III) or (ii) at least one non-aroma chemical carrier, or (iii) both (i) and (ii).

### Background

Aroma chemicals, especially fragrances, are of great interest, especially in the field of cosmetics, cleaning and laundry compositions.

Despite a large number of already existing synthetic aroma chemicals, there is a constant need for new components in order to be able to satisfy the multitude of properties desired for extremely diverse areas of application. These include, firstly, the sensory properties, i.e. the compounds should have advantageous odiferous (olfactory) properties. Furthermore, aroma chemicals should also have additional positive secondary properties, such as e.g. an efficient preparation method, the possibility of providing better sensory profiles as a result of synergistic effects with other aroma chemicals, a higher stability under certain application conditions, and/or a higher substantivity.

Such properties are of special interest for compositions such as for example, care compositions, hygiene articles, cleaning compositions, textile detergent compositions and compositions for scent dispensers.

Of special interest are aroma chemicals, which can impart one or more distinct sensory impressions to a composition, thereby contributing to a rich and interesting sensory profile, especially an olfactory profile of the composition. In this regard, aroma chemicals which can impart a Agrumic note, Chrysanthemum note, Floral note, Muguet note, Watery note, Waxy note, Citric note, Lemon note, Aldehydic note, Green note or a combination of two or more notes are of major interest. In addition, the substantivity as well as the tenacity are of special interest in order to obtain a long-lasting odiferous impression in the composition as well as to the surface which is treated with the composition.

However, since even small changes in chemical structure bring about massive changes in the sensory properties such as odor and/or flavor, the targeted search for substances with certain and distinct sensory properties such as a certain odor is extremely difficult. The search for new aroma chemicals is therefore in most cases difficult and laborious without knowing whether a substance with the desired odor and/or flavor will even actually be found.

It is an object of the presently claimed invention to provide substances which can be used as an aroma chemical either alone or as mixtures in compositions, in particular odor-intensive substances having a pleasant odor are sought. Furthermore, they should be combinable with other aroma chemicals, allowing the creation of novel advantageous sensory profiles and can be used in compositions.

It is an object of the presently claimed invention to provide a new aroma chemical or mixture of aroma chemicals which has pleasant olfactory impression; preferably a combination of two or more of the impression which are selected from the group consisting of Agrumic note, Chrysanthemum note, Floral note, Muguet note, Watery note, Waxy note, Citric note, Lemon note, Aldehydic note, Green note or a combination of two or more notes.

A further object of the present invention is that the aroma chemicals should be obtainable from readily available starting materials, allowing their fast and economic manufacturing.

### Summary

These objects were achieved by the provision of inventive mixture of the present invention. It was surprisingly found that the mixtures of the present invention had pleasant olfactory impression; preferably a combination of two or more of the impression which are selected from Agrumic note, Chrysanthemum note, Floral note, Muguet note, Watery note, Waxy note, Citric note, Lemon note, Aldehydic note, Green note.

A first aspect of the presently claimed invention relates to the use of the mixture (Mixture I) comprising at least one compound of formula (I) 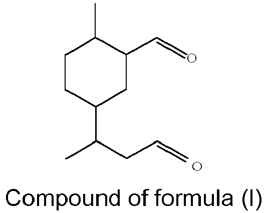
or stereoisomer thereof
and at least one compound of formula (II) 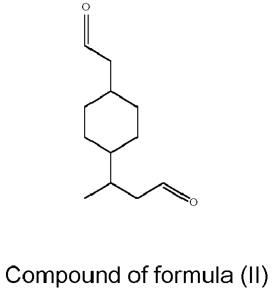
or stereoisomer thereof,
to impart an aroma impression to a composition.

A second aspect of the presently claimed invention relates to the use of the mixture (Mixture II) comprising at least one compound of formula (I) 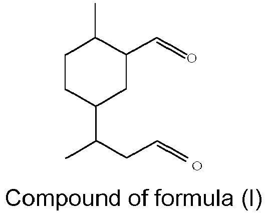
or stereoisomer thereof,
at least one compound of formula (II) 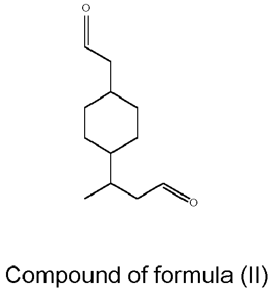
or stereoisomer thereof, and
and at least one compound of formula (III) 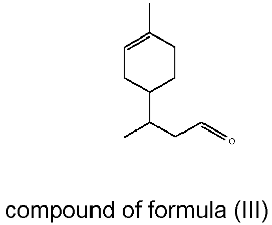
or stereoisomer thereof
to impart an aroma impression to a composition.

A further aspect of the presently claimed invention relates to a method of imparting an aroma impression to a composition comprising at least the step of adding the aforesaid mixture(s) (Mixture I or Mixture II) to a composition.

Another aspect of the presently claimed invention relates to the use the aforesaid mixture(s) (Mixture I or Mixture II) for modifying the aroma character of a composition.

A further aspect of the presently claimed invention relates to the use the aforesaid mixture(s) (Mixture I or Mixture II) to impart a Agrumic note, Chrysanthemum note, Floral note, Muguet note, Watery note, Waxy note, Citric note, Lemon note, Aldehydic note, Green note, or any combination of two or more of these to a composition.

A further aspect of the presently claimed invention relates to a method of boosting the aroma of a composition. Said method comprises the step of mixing the mixture with other ingredients such as, e.g., at least one other aroma chemical different from compound of formula (I), (II) and (III); and/or at least one non-aroma chemical carrier so as to obtain the aroma composition.

Yet another aspect of the presently claimed invention relates to a method of modifying the aroma of a composition. Said method comprises the step of incorporating at least Mixture I or Mixture II of the presently claimed invention into an aroma composition so as to obtain an aroma-modified aroma composition.

Furthermore, compound of formula (I), compound of formula (II), compound of formula (III) or mixtures thereof, can be produced in good yields and purities by a simple synthesis starting from readily available starting materials and can be produced in large scales and in a simple and cost-efficient manner.

### Detailed description

Reference throughout this specification to "one embodiment" or "an embodiment" or "preferred embodiment" means that a particular feature, structure or characteristic described in connection with the embodiment is included in at least one embodiment of the presently claimed invention. Thus, appearances of the phrases "in one embodiment" or "In a preferred embodiment" or "in a preferred embodiment" in various places throughout this specification are not necessarily all referring to the same embodiment but may refer. Furthermore, the features, structures or characteristics may be combined in any suitable manner, as would be apparent to a person skilled in the art from this disclosure, in one or more embodiments. Furthermore, while some embodiments described herein include some, but not other features included in other embodiments, combinations of features of different embodiments are meant to be within the scope of the subject matter, and form different embodiments, as would be understood by those in the art. For example, in the appended claims, any of the claimed embodiments are used in any combination.

Furthermore, the ranges defined throughout the specification include the end values as well, i.e. a range of 1 to 10 implies that both 1 and 10 are included in the range. For the avoidance of doubt, the applicant shall be entitled to any equivalents according to applicable law.

In the context of the present invention, the term "aroma" refers to a sensory property and comprises an odor and/or a flavor.

The term "aroma chemical" denotes a substance which is used to obtain a sensory or organoleptic (used interchangeably herein) impression and comprises its use to obtain an olfactory and/or a flavor impression. The term "olfactory impression" or "note" (used interchangeably here) denotes an odor impression without any positive or negative judgement, while the term "scent impression" or "fragrance impression" or "aroma impression" (used interchangeably herein) as used herein is connected to an odor impression which is generally felt as pleasant. Thus a "fragrance" or "scent" denotes an aroma chemical, which predominately induces a pleasant odor impression. A flavor denotes an aroma chemical, which induces a taste impression.

The term "aroma composition", as used herein, refers to a composition which induces an aroma. The term aroma composition comprises "odor composition" and/or "flavor composition". An odor composition being a composition, which predominately induces an odor impression, a flavor composition being a composition, which predominantly induces a taste impression.

The term "aroma profile" denotes the overall aroma impression of an aroma chemical and is composed of the individual aroma impressions of an aroma chemical.

The term odor composition comprises "fragrance composition" or "scent composition" (used interchangeably herein), which predominately induce an odor impression which is generally felt as pleasant.

The general expressions "advantageous sensory properties" or "advantageous organoleptic properties" describe the niceness and conciseness of an organoleptic impression conveyed by an aroma chemical. "Niceness" and "conciseness" are terms which are familiar to the person skilled in the art, such as a perfumer. Niceness generally refers to a spontaneously brought about, positively perceived, pleasant sensory impression. However, "nice" does not have to be synonymous with "sweet". "Nice" can also be the odor of musk or sandalwood. "Conciseness" generally refers to a spontaneously brought about sensory impression which - for the same test panel - brings about a reproducibly identical reminder of something specific. For example, a substance can have an odor which is spontaneously reminiscent of that of an "apple": the odor would then be concisely of "apples". If this apple odor were very pleasant because the odor is reminiscent, for example, of a sweet, fully ripe apple, the odor would be termed "nice". However, the odor of a typically tart apple can also be concise. If both reactions arise upon smelling the substance, in the example thus a nice and concise apple odor, then this substance has particularly advantageous sensory properties.

The expressions "combination of", "in combination with" or "combined with" when used herein referring to the compositions, methods or the use of two compounds, take account of the fact that the two compounds do not need to be used in the form of a physical mixture of said compounds but can be used (e.g., added) separately. Where the compounds are used separately, they can be used (e.g. added) sequentially (i.e. one after the other) in any order, or concurrently (i.e. basically at the same time).

The term "boosting", or "boost" is used herein to describe the effect of enhancing and/or modifying the aroma of an aroma chemical or of a composition, respectively of an aroma composition. The term "enhancing" comprises an improvement of the niceness and/or conciseness of an aroma and/or an improvement of the intensity. The term "modifying" comprises the change of an aroma profile. The terms "niceness" and "conciseness" are familiar to the person skilled in the art, such as a perfumer and have the respective meaning.

The intensity can be determined via a threshold value determination. A threshold value of an odor is the concentration of a substance in the relevant gas space at which an odor impression can just still be perceived by a representative test panel, although it no longer has to be defined.

Booster effects are particularly desired in fragrance composition when top-note-characterized applications are required, in which the odor is to be conveyed particularly quickly and intensively, for example in deodorants, air fresheners or in the taste sector in chewing gums.

The terms "the invention relates to" and "the invention is directed to" are used synonymously throughout the invention.

The term "tenacity" describes the evaporation behavior over time of an aroma chemical. The tenacity can for example be determined by applying the aroma chemical to a test strip, and by subsequent olfactory evaluation of the odor impression of the test strip. For aroma chemicals with high tenacity the time span after which the panel can still identify an aroma impression is long.

The term "substantivity" describes the interaction of an aroma chemical with a surface, such as for example the skin or a textile, especially after subsequent treatment of the surface, such as for example washing. The substantivity can for example be determined by washing a textile with a textile detergent composition comprising the aroma chemical and subsequent olfactory evaluation of the textile directly after washing (wet textile) as well as evaluation of the dry textile after prolonged storage.

The term "stability" describes the behavior of an aroma chemical upon contact with oxygen, light and/or other substances. An aroma chemical with high stability maintains its aroma profile over a long period in time, preferably in a large variety of compositions and under various storage conditions.

In order to impart a long-lasting aroma impression to a composition or to a surface treated with a composition, the tenacity, the substantivity as well as the stability of the aroma chemical in the compositions should preferably be high.

As used herein the terms "compound (I)" or "compounds (I)" refer to the compound(s) of formula (I) including, all the stereoisomeric forms (stereoisomers) thereof in all ratios and the salts thereof. The term compound (I) or compound of formula(I) is used interchangeably. This applies to compound of formula (II) and compound of formula (III).

The term "Mixture I" used herein denotes a mixture of at least one compound of formula (I) or its stereoisomers and at least one compound of formula (II) or its stereoisomers. Similarly, the term "Mixture II" used herein, denotes, a mixture of at least one compound of formula (I) or its stereoisomers, at least one compound of formula (II) or its stereoisomers and at least one compound of formula (III) or its stereoisomers.

The term "stereoisomer" is a general term used for all isomers of individual compounds that differ only in the orientation of their atoms in space, not in the connectivity of the atoms Thus, the term stereoisomer includes mirror image isomers (enantiomers), geometric (cis/trans or E/Z) isomers, and diastereoisomers. For precise definitions of the terms, see G. Helmchen:"Vocabulary and Nomenclature of Organic Stereochemistry", in Houben-Weyl E21a, Stereoselective Synthesis G. Helmchen, R.W. Hoffmann, J. Mulzer, E. Schaumann (Hrsg.), 1995, 1-74. The possible isomers can be present as mixtures (i.e. racemates, cis/trans-mixtures or mixtures of diasteroisomers).

Mixture:
Mixture I:
   One embodiment of the presently claimed invention relates to the use of a mixture comprising at least one compound of formula (I) 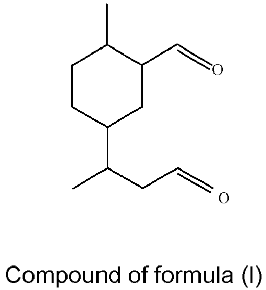
      or stereoisomer thereof,
      and at least one compound of formula (II) 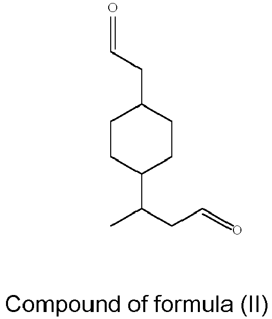
      or stereoisomer thereof,
      to impart an aroma impression to a composition.
   In a preferred embodiment, the mixture comprises compounds of formula (I) and compounds of formula (II) in a weight ratio in the range 0.01:99.99 to 99.99:0.01.
   In a more preferred embodiment, the mixture comprises compounds of formula (I) and compounds of formula (II) in a weight ratio in the range of 10:90 to 90:10.
   In yet another preferred embodiment, the mixture comprises compounds of formula (I) and compounds of formula (II) in a weight ratio in the range of 20:80 to 80:20, preferably in the range 25:75 to75:25.
   In an embodiment, the mixture of at least one compound of formula I and at least one compound of formula II can be obtained directly from the reaction mixture or can be physically mixed in any desired ratio depending on the end use of the combination in any suitable composition.
Mixture II:
   In a further embodiment the mixture of compound of formula (I) and compound of formula (III) further comprises at least one compound of formula (III).

In an embodiment, the presently claimed invention relates to a mixture comprising at least one compound of formula (I), at least one compound of formula (II) and at least one compound of formula (III) 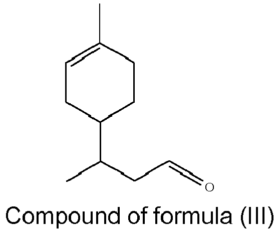 or stereoisomer thereof.
to impart an aroma impression to a composition.

In a preferred embodiment, the mixture comprises compound of formula (I) compound of formula (II) and compound of formula (III) in a weight ratio in the range (I: II: III) of 0.5-80:0.1-15:0.1-99, respectively.

In a preferred embodiment the mixture comprises compound of formula (I) in an amount which ranges from 0.5 to 80 which includes all the fractional numbers within this range.

In a preferred embodiment the mixture comprises compound of formula (I) in an amount which ranges from 0.1 to 15 which includes all the fractional numbers within this range.

In a preferred embodiment the mixture comprises compound of formula (I) in an amount which ranges from 0.1 to 99 which includes all the fractional numbers within this range.

In a preferred embodiment, the mixture comprises compound of formula (I) compound of formula (II) and compound of formula (III) in a weight ratio in the range (I: II: III) of 0.05-15:0.05-15:0.1-99.9, respectively.

In a preferred embodiment, the mixture comprises compound of formula (I) compound of formula (II) and compound of formula (III) in a weight ratio in the range (I: II: III) of 0.1-10:0.1-10:0.1-99.8, respectively.

In another preferred embodiment, the mixture comprises compounds of formula (I) compounds of formula (II) and compounds of formula (III) in a weight ratio of 0.8:0.1:99 respectively.

In another preferred embodiment, the mixture comprises compounds of formula (I) compounds of formula (II) and compounds of formula (III) in a weight ratio of 0.05:0.05:99.9 respectively.

By varying the amount of compound of formula (III) in the mixtures the aroma impression changes significantly. A small amount of compound of formula (III) in the mixture, changes the sensory properties of the mixtures. This subtle change in the mixture composition gives rise to several possible aroma impressions of varying degree and hence can be used as aroma imparting compositions in several applications.

In an embodiment, the mixture of at least one compound of formula (I), at least one compound of formula (II), and at least one compound of formula (III), can be obtained directly from the reaction mixture or can be physically mixed in any desired ratio depending on the end use of the combination in any suitable composition.

A preferred embodiment of the presently claimed invention is direct to the use of Mixture I to impart Agrumic note, Chrysanthemum note, Floral note, Muguet note, Watery note, Waxy note or a combination of two or more notes of these notes to a composition.

Another preferred embodiment of the presently claimed invention is direct to the use of Mixture II to impart Agrumic note, Chrysanthemum note, Floral note, Muguet note, Watery note, Waxy note, Citric note, Lemon note, Aldehydic note, Green note or a combination of two or more notes to a composition.

In a further embodiment the presently claimed invention is directed to the use of the Mixture I or Mixture II to boost the aroma of a composition.

In an embodiment of the presently claimed invention, Mixture I or Mixture II is used as a fragrance or as constituent of a fragrance composition.

Suitable compositions are for example compositions used in personal care, in home care, in industrial applications as well as compositions used in other applications, such as pharmaceutical compositions or crop protection compositions.

Preferably, the mixtures according to the presently claimed invention are used in a composition selected from perfume compositions, body care compositions (including cosmetic compositions and products for oral and dental hygiene), hygiene articles, cleaning compositions (including dishwashing compositions), textile detergent compositions, compositions for scent dispensers, foods, food supplements, pharmaceutical compositions, or crop protection compositions.

The compositions have been described in the below paragraphs.

Composition:
In an embodiment, the presently claimed invention relates to a composition comprising Mixture I or Mixture II, and
(i) at least one aroma chemical (X) other than compound of formula (I), compound of formula (II), and compound of formula (III) or stereoisomers thereof, or
(ii) at least one non-aroma chemical carrier, or
(iii) both of (i) and (ii).

Preferably in this embodiment, the composition comprises the Mixture I or Mixture II, in a total amount in the range of ≥ 0.01 wt.% to ≤ 70.0 wt.%, based on the total weight of the composition.

Preferably, the composition is an aroma composition, more preferable a fragrance composition.

Aroma chemical different from compound of formula (I), compound of formula (II), compound of formula (III).

In one embodiment, the composition comprises at least one aroma chemical which is different from compound of formula (I), compound of formula (II) and compound of formula (III). Aroma chemicals which are different from compound of formula (I), (II) and (III) are also referred to as aroma chemical (X).

By virtue of their physical properties, Mixture I or Mixture II is well combinable with aroma chemicals which are different from compound of formula (I), compound of formula (II) and compound of formula (III) and other customary ingredients in aroma compositions, in particular fragrance compositions. This allows, e.g., the creation of aroma compositions (preferably fragrance compositions) which have novel advantageous sensory profiles. Especially, as already explained above, the compounds can provide a booster effect for other aroma chemicals (such as fragrances).

The aroma chemical (X) is preferably selected from:
geranyl acetate, alpha-hexylcinnamaldehyde, 2 phenoxyethyl isobutyrate, dihydromyrcenol, methyl dihydrojasmonate, 4,6,6,7,8,8 hexamethyl-1,3,4,6,7,8-hexa-hydro¬cyclopenta[g]benzopyran, tetrahydrolinalool, ethyllinalool, benzyl salicylate, 2 methyl-3-(4-tert-butylphenyl)propanal, cinnamyl alcohol, 4,7 methano-3a,4,5,6,7,7a-hexahydro-5 indenyl acetate and/or 4,7 methano-3a,4,5,6,7,7a-hexahydro-6-indenyl acetate, citronellol, citronellyl acetate, tetrahydrogeraniol, vanillin, linalyl acetate, styrolyl acetate, octahydro-2,3,8,8-tetramethyl-2-acetonaphthone and/or 2 acetyl-1,2,3,4,6,7,8-octahydro-2,3,8,8-tetramethylnaphthalene, hexyl salicylate, 4 tert-butylcyclohexyl acetate, 2-tert-butylcyclohexyl acetate, alpha-ionone, n alpha-methylionone, alpha-iso-methylionone, coumarin, terpinyl acetate, 2 phenylethyl alcohol, 4-(4-hydroxy-4-methylpentyl)-3-cyclohexene-carboxaldehyde, alpha-amylcinnamaldehyde, ethylene brassylate, (E) and/or (Z)-3-methylcyclopentadec-5 enone, 15-pentadec-11-enolide and/or 15-pentadec-12-enolide, 15-cyclopentadecanolide, 1-(5,6,7,8-tetrahydro-3,5,5,6,8,8-hexamethyl-2-naphthalenyl)ethanone, 2-isobutyl-4-methyltetrahydro-2H pyran-4-ol, 2-ethyl-4-(2,2,3-trimethyl-3-cyclopenten-1-yl)-2-buten-1-ol, cis-3-hexenyl acetate, trans-3-hexenyl acetate, trans-2/cis-6-nonadienol, 2,4-dimethyl-3-cyclohexenecarboxaldehyde, 2,4,4,7-tetramethyloct-6-en-3-one, 2,6-dimethyl-5-hepten-1-al, borneol, 3 (3 isopropylphenyl)butanal, 2-methyl-3-(3,4-methylenedioxyphenyl)propanal, 3-(4-ethylphenyl)-2,2-dimethylpropanal, 7-methyl-2H 1,5-benzodioxepin-3(4H)-one, 3,3,5-trimethylcyclohexyl acetate, 2,5,5 trimethyl-1,2,3,4,4a,5,6,7-octahydronaphthalen-2-ol, 3-(4-tert-butylphenyl)-propanal, ethyl 2-methylpentanoate, ethoxymethoxycyclododecane, 2,4-dimethyl-4,4a,5,9b-tetrahydroindeno[1,2-d][1,3]dioxine, (2-tert-butylcyclohexyl) acetate, or 3-[5,5,6-trimethylbicyclo[2.2.1]hept-2-yl]cyclohexan-1-ol.

In a preferred embodiment, the at least one aroma chemical (X) is selected from methyl benzoate, benzyl acetate, geranyl acetate, 2-isobutyl-4-methyltetrahydro-2H-pyran-4-ol, linalool, 2-isobutyl-4-methyltetrahydro-2H-pyran-4-ol, or methyl benzoate.

In another preferred embodiment, the at least one aroma chemical (X) is selected from ethylvanillin, vanillin, 2,5-dimethyl-4-hydroxy-2H-furan-3-one (furaneol), or 3-hydroxy-2-methyl-4H-pyran-4-one (maltol).

Further aroma chemicals with which the compound of formula (I) can be combined to give a composition according to the presently claimed invention can be found, e.g., in S. Arctander, Perfume and Flavor Chemicals, Vol. I and II, Montclair, N. J., 1969, self-published or H. Surburg and J. Panten, Common Fragrance and Flavor Materials, 4th Ed., Wiley- VCH, Weinheim 2016. Specifically, mention may be made of:
extracts from natural raw materials such as essential oils, concretes, absolutes, resins, resinoids, balsams, tinctures such as e.g.
ambergris tincture; amyris oil; angelica seed oil; angelica root oil; aniseed oil; valerian oil; basil oil; tree moss absolute; bay oil; mugwort oil; benzoin resin; bergamot oil; beeswax absolute; birch tar oil; bitter almond oil; savory oil; buchu leaf oil; cabreuva oil; cade oil; calmus oil; camphor oil; cananga oil; cardamom oil; cascarilla oil; cassia oil; cassia absolute; castoreum absolute; cedar leaf oil; cedar wood oil; cistus oil; citronella oil; lemon oil; copaiba balsam; copaiba balsam oil; coriander oil; costus root oil; cumin oil; cypress oil; davana oil; dill weed oil; dill seed oil; Eau de brouts absolute; oak moss absolute; elemi oil; tarragon oil; eucalyptus citriodora oil; eucalyptus oil; fennel oil; pine needle oil; galbanum oil; galbanum resin; geranium oil; grapefruit oil; guaiacwood oil; gurjun balsam; gurjun balsam oil; helichrysum absolute; helichrysum oil; ginger oil; iris root absolute; iris root oil; jasmine absolute; calmus oil; camomile oil blue; roman camomile oil; carrot seed oil; cascarilla oil; pine needle oil; spearmint oil; caraway oil; labdanum oil; labdanum absolute; labdanum resin; lavandin absolute; lavandin oil; lavender absolute; lavender oil; lemongrass oil; lovage oil; lime oil distilled; lime oil pressed; linalool oil; litsea cubeba oil; laurel leaf oil; mace oil; marjoram oil; mandarin oil; massoia bark oil; mimosa absolute; musk seed oil; musk tincture; clary sage oil; nutmeg oil; myrrh absolute; myrrh oil; myrtle oil; clove leaf oil; clove flower oil; neroli oil; olibanum absolute; olibanum oil; opopanax oil; orange blossom absolute; orange oil; origanum oil; palmarosa oil; patchouli oil; perilla oil; peru balsam oil; parsley leaf oil; parsley seed oil; petitgrain oil; peppermint oil; pepper oil; pimento oil; pine oil; pennyroyal oil; rose absolute; rose wood oil; rose oil; rosemary oil; Dalmatian sage oil; Spanish sage oil; sandalwood oil; celery seed oil; spike-lavender oil; star anise oil; styrax oil; tagetes oil; fir needle oil; tea tree oil; turpentine oil; thyme oil; tolubalsam; tonka absolute; tuberose absolute; vanilla extract; violet leaf absolute; verbena oil; vetiver oil; juniper berry oil; wine lees oil; wormwood oil; winter green oil; hyssop oil; civet absolute; cinnamon leaf oil; cinnamon bark oil, and fractions thereof, or ingredients isolated therefrom;
individual fragrances from the group of hydrocarbons, such as e.g. 3 carene; alpha-pinene; beta-pinene; alpha-terpinene; gamma-terpinene; p-cymene; bisabolene; camphene; caryophyllene; cedrene; farnesene; limonene; longifolene; myrcene; ocimene; valencene; (E,Z)-1,3,5-undecatriene; styrene; diphenylmethane;
the aliphatic alcohols such as e.g. hexanol; octanol; 3-octanol; 2,6-dimethylheptanol; 2-methyl-2-heptanol; 2-methyl-2-octanol; (E)-2-hexenol; (E)- and (Z)-3-hexenol; 1 octen-3-ol; mixture of 3,4,5,6,6-pentamethyl-3/4-hepten-2-ol and 3,5,6,6-tetramethyl-4-methyleneheptan-2-ol; (E,Z)-2,6-nonadienol; 3,7-dimethyl-7-methoxyoctan-2-ol; 9-decenol; 10-undecenol; 4-methyl-3-decen-5-ol;
the aliphatic aldehydes and acetals thereof such as e.g. hexanal; heptanal; octanal; nonanal; decanal; undecanal; dodecanal; tridecanal; 2-methyloctanal; 2-methylnonanal; (E)-2-hexenal; (Z)-4-heptenal; 2,6-dimethyl-5-heptenal; 10-undecenal; (E)-4-decenal; 2-dodecenal; 2,6,10-trimethyl-9-undecenal; 2,6,10 trimethyl-5,9-undecadienal; heptanal diethylacetal; 1,1-dimethoxy-2,2,5 trimethyl-4-hexene; citronellyloxyacetaldehyde; (E/Z)-1-(1-methoxypropoxy)-hex-3-ene; the aliphatic ketones and oximes thereof such as e.g. 2-heptanone; 2-octanone; 3-octanone; 2-nonanone; 5-methyl-3-heptanone; 5-methyl-3 heptanone oxime; 2,4,4,7-tetramethyl-6-octen-3-one; 6-methyl-5-hepten-2-one;
the aliphatic sulfur-containing compounds such as e. g. 3-methylthiohexanol; 3-methylthiohexyl acetate; 3-mercaptohexanol; 3-mercaptohexyl acetate; 3-mercaptohexyl butyrate; 3-acetylthiohexyl acetate; 1-menthene-8-thiol;
the aliphatic nitriles such as e. g. 2-nonenenitrile; 2-undecenenitrile; 2 tridecenenitrile; 3,12-tridecadienenitrile; 3,7-dimethyl-2,6-octadienenitrile; 3,7-dimethyl-6 octenenitrile;
the esters of aliphatic carboxylic acids such as e.g. (E) and (Z)-3-hexenyl formate; ethyl acetoacetate; isoamyl acetate; hexyl acetate; 3,5,5-trimethylhexyl acetate; 3 methyl-2-butenyl acetate; (E)-2-hexenyl acetate; (E) and (Z)-3-hexenyl acetate; octyl acetate; 3-octyl acetate; 1-octen-3-yl acetate; ethyl butyrate; butyl butyrate; isoamyl butyrate; hexyl butyrate; (E) and (Z)-3-hexenyl isobutyrate; hexyl crotonate; ethyl isovalerate; ethyl 2-methylpentanoate; ethyl hexanoate; allyl hexanoate; ethyl heptanoate; allyl heptanoate; ethyl octanoate; ethyl (E,Z)-2,4-decadienoate; methyl 2-octinate; methyl 2-noninate; allyl 2-isoamyloxy acetate; methyl-3,7-dimethyl-2,6-octadienoate; 4-methyl-2-pentyl crotonate;
the acyclic terpene alcohols such as e.g. geraniol; nerol; linalool; lavandulol; nerolidol; farnesol; tetrahydrolinalool; 2,6-dimethyl-7-octen-2-ol; 2,6-dimethyloctan-2-ol; 2-methyl-6-methylene-7-octen-2-ol; 2,6-dimethyl-5,7-octadien-2-ol; 2,6-dimethyl-3,5-octadien-2 ol; 3,7-dimethyl-4,6-octadien-3-ol; 3,7-dimethyl-1 ,5,7-octatrien-3-ol; 2,6-dimethyl-2,5,7-octatrien-1-ol; and the formates, acetates, propionates, isobutyrates, butyrates, isovalerates, pentanoates, hexanoates, crotonates, tiglinates and 3-methyl-2 butenoates thereof;
the acyclic terpene aldehydes and ketones such as e.g. geranial; neral; citronellal; 7 hydroxy-3,7-dimethyloctanal; 7 methoxy-3,7-dimethyloctanal; 2,6,10-trimethyl-9 undecenal; geranyl acetone; as well as the dimethyl and diethylacetals of geranial, neral, 7-hydroxy-3,7-dimethyloctanal; the cyclic terpene alcohols such as e.g. menthol; isopulegol; alpha-terpineol; terpine-4-ol; menthan-8-ol; menthan-1-ol; menthan-7-ol; borneol; isoborneol; linalool oxide; nopol; cedrol; ambrinol; vetiverol; guajol; and the formates, acetates, propionates, isobutyrates, butyrates, isovalerates, pentanoates, hexanoates, crotonates, tiglinates and 3-methyl-2-butenoates thereof;
the cyclic terpene aldehydes and ketones such as e.g. menthone; isomenthone; 8 mercaptomenthan-3-one; carvone; camphor; fenchone; alpha-ionone; beta-ionone; alpha-n-methylionone; beta-n-methylionone; alpha-isomethylionone; beta-isomethylionone; alpha-irone; alpha-damascone; beta-damascone; beta-damascenone; delta-damascone; gamma-damascone; 1-(2,4,4-trimethyl-2-cyclohexen-1-yl)-2-buten-1-one; 1,3,4,6,7,8a-hexahydro-1,1,5,5-tetramethyl-2H-2,4a-methano¬naphthalene-8(5H)-one; 2-methyl-4-(2,6,6-trimethyl-1-cyclohexen-1-yl)-2-butenal; nootkatone; dihydronootkatone; 4,6,8-megastigmatrien-3-one; alpha-sinensal; beta-sinensal; acetylated cedar wood oil (methyl cedryl ketone);
the cyclic alcohols such as e.g. 4-tert-butylcyclohexanol; 3,3,5-trimethylcyclohexanol; 3-isocamphylcyclohexanol; 2,6,9-trimethyl-Z2,Z5,E9-cyclododecatrien-1-ol; 2-isobutyl-4-methyltetrahydro-2H-pyran-4-ol;
the cycloaliphatic alcohols such as e.g. alpha-3,3-trimethylcyclohexylmethanol; 1 (4-isopropylcyclohexyl)ethanol; 2-methyl-4-(2,2,3-trimethyl-3-cyclopent-1-yl)butanol; 2-methyl-4-(2,2,3 trimethyl-3-cyclopent-1-yl)-2-buten-1-ol; 2-ethyl-4-(2,2,3-trimethyl-3 cyclopent-1-yl)-2-buten-1-ol; 3-methyl-5-(2,2,3 trimethyl-3-cyclopent-1-yl)pentan-2 ol; 3-methyl-5-(2,2,3-trimethyl-3-cyclopent-1-yl)-4-penten-2-ol; 3,3-dimethyl-5-(2,2,3-trimethyl-3-cyclopent-1-yl)-4-penten-2-ol; 1-(2,2,6-trimethylcyclohexyl)pentan-3-ol; 1-(2,2,6-trimethylcyclohexyl)hexan-3-ol;
the cyclic and cycloaliphatic ethers such as e.g. cineol; cedryl methyl ether; cyclododecyl methyl ether; 1,1-dimethoxycyclododecane; (ethoxymethoxy)cyclo-dodecane; alpha-cedrene epoxide; 3a,6,6,9a-tetramethyldodecahydronaphtho[2,1-b]furan; 3a-ethyl-6,6,9a-trimethyldodecahydro-naphtho[2,1-b]furan; 1,5,9-trimethyl-13-oxabicyclo-[10.1.0]trideca-4,8-diene; rose oxide; 2-(2,4-dimethyl-3-cyclohexen-1-yl)-5-methyl-5-(1-methylpropyl)-1,3-dioxane;
the cyclic and macrocyclic ketones such as e.g. 4-tert-butylcyclohexanone; 2,2,5 trimethyl-5-pentylcyclopentanone; 2-heptylcyclopentanone; 2-pentylcyclo-pentanone; 2-hydroxy-3-methyl-2-cyclopenten-1-one; 3-methyl-cis-2-penten-1-yl-2 cyclopenten-1-one; 3-methyl-2-pentyl-2-cyclopenten-1-one; 3-methyl-4-cyclopenta-decenone; 3-methyl-5-cyclopentadecenone; 3-methylcyclopentadecanone; 4-(1-ethoxyvinyl)-3,3,5,5-tetramethylcyclohexanone; 4-tert-pentylcyclohexanone; 5-cyclohexadecen-1-one; 6,7-dihydro-1,1,2,3,3-pentamethyl-4(5H)-indanone; 8-cyclo-hexadecen-1-one; 7-cyclohexadecen-1-one; (7/8)-cyclohexadecen-1-one; 9 cyclo-heptadecen-1-one; cyclopentadecanone; cyclohexadecanone;
the cycloaliphatic aldehydes such as e. g. 2,4-dimethyl-3-cyclohexenecarbaldehyde; 2 methyl-4-(2,2,6-trimethylcyclohexen-1-yl)-2-butenal; 4-(4-hydroxy-4-methylpentyl)-3 cyclohexene carbaldehyde; 4-(4-methyl-3-penten-1-yl)-3-cyclohexenecarbaldehyde;
the cycloaliphatic ketones such as e. g. 1-(3,3-dimethylcyclohexyl)-4-penten-1-one; 2,2 dimethyl-1-(2,4-dimethyl-3-cyclohexen-1-yl)-1-propanone; 1-(5,5-dimethyl-1 cyclo-hexen-1-yl)-4-penten-1-one; 2,3,8,8-tetramethyl-1,2,3,4,5,6,7,8-octahydro-2-naphthalenyl methyl ketone; methyl 2,6,10-trimethyl-2,5,9-cyclododecatrienyl ketone; tert-butyl (2,4-dimethyl-3-cyclohexen-1-yl) ketone;
the esters of cyclic alcohols such as e.g. 2-tert-butylcyclohexyl acetate; 4-tert-butylcyclohexyl acetate; 2-tert-pentylcyclohexyl acetate; 4-tert-pentylcyclohexyl acetate; 3,3,5-trimethylcyclohexyl acetate; decahydro-2-naphthyl acetate; 2-cyclopentylcyclopentyl crotonate; 3-pentyltetrahydro-2H-pyran-4-yl acetate; decahydro-2,5,5,8a-tetramethyl-2-naphthyl acetate; 4,7-methano-3a,4,5,6,7,7a-hexahydro-5 or 6-indenyl acetate; 4,7-methano-3a,4,5,6,7,7a-hexahydro-5 or 6 indenyl propionate; 4,7-methano-3a,4,5,6,7,7a-hexahydro-5 or 6-indenyl isobutyrate; 4,7 methanooctahydro-5 or 6-indenyl acetate;
the esters of cycloaliphatic alcohols such as e.g. 1-cyclohexylethyl crotonate;
the esters of cycloaliphatic carboxylic acids such as e.g. allyl 3-cyclohexylpropionate; allyl cyclohexyloxyacetate; cis and trans-methyl dihydrojasmonate; cis and trans-methyl jasmonate; methyl 2-hexyl-3-oxocyclopentanecarboxylate; ethyl 2-ethyl-6,6 dimethyl-2-cyclohexenecarboxylate; ethyl 2,3,6,6-tetramethyl-2 cyclohexene-carboxylate; ethyl 2-methyl-1,3-dioxolane-2-acetate;
the araliphatic alcohols such as e.g. benzyl alcohol; 1-phenylethyl alcohol, 2 phenylethyl alcohol, 3-phenylpropanol; 2-phenylpropanol; 2-phenoxyethanol; 2,2-dimethyl-3-phenylpropanol; 2,2-dimethyl-3-(3-methylphenyl)propanol; 1,1-dimethyl-2 phenylethyl alcohol; 1,1-dimethyl-3-phenylpropanol; 1-ethyl-1-methyl-3-phenylpropanol; 2-methyl-5-phenylpentanol; 3-methyl-5-phenylpentanol; 3-phenyl-2-propen-1-ol; 4-methoxy¬benzyl alcohol; 1-(4-isopropylphenyl)ethanol;
the esters of araliphatic alcohols and aliphatic carboxylic acids such as e.g. benzyl acetate; benzyl propionate; benzyl isobutyrate; benzyl isovalerate; 2-phenylethyl acetate; 2-phenylethyl propionate; 2-phenylethyl isobutyrate; 2 phenylethyl isovalerate; 1 phenylethyl acetate; alpha-trichloromethylbenzyl acetate; alpha,alpha-dimethylphenylethyl acetate; alpha,alpha-dimethylphenylethyl butyrate; cinnamyl acetate; 2-phenoxyethyl isobutyrate; 4-methoxybenzyl acetate;
the araliphatic ethers such as e.g. 2-phenylethyl methyl ether; 2 phenylethyl isoamyl ether; 2-phenylethyl 1-ethoxyethyl ether; phenylacetaldehyde dimethyl acetal; phenylacetaldehyde diethyl acetal; hydratropaaldehyde dimethyl acetal; phenylacetaldehyde glycerol acetal; 2,4,6-trimethyl-4-phenyl-1,3-dioxane; 4,4a,5,9b-tetrahydroindeno[1,2-d]-m-dioxine; 4,4a,5,9b-tetrahydro-2,4-dimethylindeno[1,2-d]-m dioxine;
the aromatic and araliphatic aldehydes such as e.g. benzaldehyde; phenylacetaldehyde; 3-phenylpropanal; hydratropaaldehyde; 4-methylbenzaldehyde; 4 methylphenylacetaldehyde; 3-(4-ethylphenyl)-2,2-dimethylpropanal; 2-methyl-3-(4-isopropylphenyl)propanal; 2-methyl-3-(4-tert-butylphenyl)propanal; 2-methyl-3-(4-isobutylphenyl)propanal; 3-(4-tert-butylphenyl)propanal; cinnamaldehyde; alpha-butylcinnamaldehyde; alpha-amylcinnamaldehyde; alpha-hexylcinnamaldehyde; 3 methyl-5-phenylpentanal; 4-methoxybenzaldehyde; 4-hydroxy-3 methoxy-benzaldehyde; 4-hydroxy-3-ethoxybenzaldehyde; 3,4-methylenedioxybenzaldehyde; 3,4-dimethoxybenzaldehyde; 2-methyl-3-(4-methoxyphenyl)propanal; 2-methyl-3-(4-methylenedioxyphenyl)propanal;
the aromatic and araliphatic ketones such as e.g. acetophenone; 4-methylacetophenone; 4-methoxyacetophenone; 4-tert-butyl-2,6-dimethylaceto-phenone; 4-phenyl-2-butanone; 4-(4-hydroxyphenyl)-2-butanone; 1-(2-naphthalenyl)-ethanone; 2-benzofuranylethanone; (3-methyl-2-benzofuranyl)ethanone; benzophenone; 1,1,2,3,3,6-hexamethyl-5-indanyl methyl ketone; 6-tert-butyl-1,1 dimethyl-4 indanyl methyl ketone; 1-[2,3-dihydro-1,1,2,6-tetramethyl-3-(1-methylethyl)-1H-5 indenyl]ethanone; 5',6',7',8'-Tetrahydro-3',5',5',6',8',8'-hexamethyl-2'-acetonaphthone;
the aromatic and aliphatic carboxylic acids and esters thereof such as e.g. benzoic acid; phenylacetic acid; methyl benzoate; ethyl benzoate; hexyl benzoate; benzyl benzoate; methyl phenylacetate; ethyl phenylacetate; geranyl phenylacetate; phenylethyl phenylacetate; methyl cinnamate; ethyl cinnamate; benzyl cinnamate; phenylethyl cinnamate; cinnamyl cinnamate; allyl phenoxyacetate; methyl salicylate; isoamyl salicylate; hexyl salicylate; cyclohexyl salicylate; cis-3-hexenyl salicylate; benzyl salicylate; phenylethyl salicylate; methyl 2,4-dihydroxy-3,6-dimethylbenzoate; ethyl 3-phenylglycidate; ethyl 3-methyl-3-phenylglycidate;
the nitrogen-containing aromatic compounds such as e.g. 2,4,6-trinitro-1,3-dimethyl-5 tert-butylbenzene; 3,5-dinitro-2,6-dimethyl-4-tert-butylacetophenone; cinnamonitrile; 3 methyl-5-phenyl-2-pentenonitrile; 3-methyl-5-phenylpentanonitrile; methyl anthranilate; methyl-N-methylanthranilate; Schiff bases of methyl anthranilate with 7 hydroxy-3,7-dimethyloctanal, 2-methyl-3-(4-tert-butylphenyl)propanal or 2,4 dimethyl-3-cyclohexenecarbaldehyde; 6-isopropylquinoline; 6-isobutylquinoline; 6-sec-butylquinoline; 2-(3-phenylpropyl)pyridine; indole; skatole; 2-methoxy-3 isopropyl-pyrazine; 2-isobutyl-3-methoxypyrazine;
the phenols, phenyl ethers and phenyl esters such as e.g. estragole; anethole; eugenol; eugenyl methyl ether; isoeugenol; isoeugenyl methyl ether; thymol; carvacrol; diphenyl ether; beta-naphthyl methyl ether; beta-naphthyl ethyl ether; beta-naphthyl isobutyl ether; 1,4-dimethoxybenzene; eugenyl acetate; 2-methoxy-4-methylphenol; 2 ethoxy-5-(1-propenyl)phenol; p-cresyl phenylacetate;
the heterocyclic compounds such as e.g. 2,5-dimethyl-4-hydroxy-2H-furan-3-one; 2 ethyl-4-hydroxy-5-methyl-2H-furan-3-one; 3-hydroxy-2-methyl-4H-pyran-4-one; 2 ethyl-3-hydroxy-4H-pyran-4-one;
the lactones such as e.g. 1,4-octanolide; 3-methyl-1,4-octanolide; 1,4-nonanolide; 1,4-decanolide; 8-decen-1,4-olide; 1,4-undecanolide; 1,4-dodecanolide; 1,5-decanolide; 1,5-dodecanolide; 4-methyl-1,4-decanolide; 1,15-pentadecanolide; cis and trans-11-pentadecen-1,15-olide; cis and trans-12-pentadecen-1,15-olide; 1,16-hexadecanolide; 9-hexadecen-1,16-olide; 10-oxa-1,16-hexadecanolide; 11-oxa-1,16-hexadecanolide; 12-oxa-1,16-hexadecanolide; ethylene 1,12-dodecanedioate; ethylene 1,13-tridecanedioate; coumarin; 2,3-dihydrocoumarin; octahydrocoumarin.

The aroma chemical (X) used in the composition are obtained from known commercial sources and procured from Germany.

In a preferred embodiment, the composition comprises Mixture I or Mixture II and at least one aroma chemical (X).

Non- aroma chemical carrier:
The non-aroma chemical carrier in the composition of the invention is preferably selected from the group consisting of surfactants, oil components antioxidants, deodorant-active agents and solvents.

Preferably the at least one non-aroma chemical carrier is a compound, a mixture of compounds or other additives, which has/have no or no noteworthy sensory properties. The non-aroma chemical carrier can serve for the dilution and/or the fixing of Mixture I or Mixture II and - optionally the at least one aroma chemical (X), as defined above, if comprised in the composition.

The non-aroma chemical carrier in the composition of the invention is preferably selected from surfactants, oil components, solvents, or any mixture of two or more of the aforementioned.

### Solvent

In the context of the presently claimed invention, a "solvent" serves for the dilution of Mixture I or Mixture II to be used according to the invention, without having its own aroma.

The amount of solvent(s) is adjusted depending on the composition and represents a routine task of the person skilled in the art.

Preferably, the solvent is present in the composition in a total amount of 0.01 wt.% to 99.0 wt.%, more preferably in a total amount of 0.05 wt.% to 95.0 wt.%, yet more preferably in a total amount of 0.1 wt.% to 80.0 wt.%, most preferably 0.1 wt.% to 70.0 wt.%, particularly in a total amount of 0.1 wt.% to 60.0 wt.%, based on the total weight of the composition.

In a preferred embodiment, the composition comprises 0.05 wt.% to 10 wt.%, more preferably 0.1 wt.% to 5 wt.%, yet more preferably 0.2 wt.% to 3 wt.% total solvent(s), based on the total weight of the composition. In yet another preferred embodiment of the invention, the composition comprises 20 wt.% to 70 wt.%, more preferably 25 wt.% to 50 wt.% of total solvent(s), based on the total weight of the composition.

Preferred solvents are selected from ethanol, isopropanol, diethylene glycol monoethyl ether, glycerol, propylene glycol, 1,2 butylene glycol, dipropylene glycol, triethyl citrate, isopropyl myristate, or any mixture of two or more of the aforementioned.

In a preferred embodiment, the composition comprises Mixture I or Mixture II and at least one solvent and optionally at least one aroma chemical (X).

### Oil component

Preferably, the total oil components are present in an amount of 0.1 to 80 wt.%, more preferably 0.5 to 70 wt.%, yet more preferably 1 to 60 wt.%, even more preferably 1 to 50 wt.%, particularly 1 to 40 wt.%, more particularly 5 to 25 wt.% and specifically 5 to 15 wt.%, based on the total weight of the composition.

Preferably the oil components are selected from Guerbet alcohols based on fatty alcohols containing 6 to 18, preferably 8 to 10, carbon atoms and other additional esters, such as myristyl myristate, myristyl palmitate, myristyl stearate, myristyl isostearate, myristyl oleate, myristyl behenate, myristyl erucate, cetyl myristate, cetyl palmitate, cetyl stearate, cetyl isostearate, cetyl oleate, cetyl behenate, cetyl erucate, stearyl myristate, stearyl palmitate, stearyl stearate, stearyl isostearate, stearyl oleate, stearyl behenate, stearyl erucate, isostearyl myristate, isostearyl palmitate, isostearyl stearate, isostearyl isostearate, isostearyl oleate, isostearyl behenate, isostearyl oleate, oleyl myristate, oleyl palmitate, oleyl stearate, oleyl isostearate, oleyl oleate, oleyl behenate, oleyl erucate, behenyl myristate, behenyl palmitate, behenyl stearate, behenyl isostearate, behenyl oleate, behenyl behenate, behenyl erucate, erucyl myristate, erucyl palmitate, erucyl stearate, erucyl isostearate, erucyl oleate, erucyl behenate and erucyl erucate. Also suitable are esters of C18-C38 alkyl-hydroxycarboxylic acids with linear or branched C6-C22 fatty alcohols, more especially dioctyl malate, esters of linear and/or branched fatty acids with polyhydric alcohols (for example propylene glycol, dimer dial or trimer triol), triglycerides based on C6-C10 fatty acids, liquid mono-, di- and triglyceride mixtures based on C6-C18 fatty acids, esters of C6-C22 fatty alcohols and/or Guerbet alcohols with aromatic carboxylic acids, more particularly benzoic acid, esters of dicarboxylic acids with polyols containing 2 to 10 car- bon atoms and 2 to 6 hydroxyl groups, vegetable oils, branched primary alcohols, substituted cyclohexanes, linear and branched C6-C22 fatty alcohol carbonates such as, for example, dicaprylyl carbonate (Cetiol^{®} CC), Guerbet carbonates based on fatty alcohols containing 6 to 18, preferably 8 to 10, carbon atoms, esters of benzoic acid with linear and/or branched C6 to C22 alcohols (for example Finsolv^{®} TN), linear or branched, symmetrical or nonsymmetrical dialkyl ethers containing 6 to 22 carbon atoms per alkyl group such as, for example, dicaprylyl ether (Cetiol^{®} OE), ring opening products of epoxidized fatty acid esters with polyols and hydrocarbons, or mixtures thereof.

In a preferred embodiment, the composition comprises Mixture I or Mixture II and at least one oil component and optionally at least one aroma chemical (X).

### Antioxidants

It is to be understood that antioxidants are able to inhibit or prevent the undesired changes in the compositions to be protected caused by oxygen effects and other oxidative processes. The effect of the antioxidants consists in most cases in them acting as free-radical scavengers for the free radicals which arise during autoxidation.

In a preferred embodiment, the antioxidant is selected from
- amino acids (for example glycine, alanine, arginine, serine, threonine, histidine, tyrosine, tryptophan) and derivatives thereof,
- imidazoles (e.g. urocanic acid) and derivatives thereof,
- peptides, such as D,L-carnosine, D-carnosine, L-carnosine (=β-Alanyl-L-histidine) and derivatives thereof,
- carotenoids, carotenes (e.g. alpha-carotene, beta-carotene, lycopene, lutein) or derivatives thereof,
- chlorogenic acid and derivatives thereof,
- lipoic acid and derivatives thereof (for example dihydrolipoic acid),
- auro-thioglucose, propylthiouracil and other thiols (for example thioredoxin, glutathione, cysteine, cystine, cystamine and the glycosyl, N-acetyl, methyl, ethyl, propyl, amyl, butyl and lauryl, palmitoyl, oleyl, gamma-linoleyl, cholesteryl and glyceryl esters thereof) and salts thereof,
- dilauryl thiodipropionate, distearyl thiodipropionate, thiodipropionic acid and derivatives thereof (esters, ethers, peptides, lipids, nucleotides, nucleosides and salts),
- sulfoximine compounds (for example buthionine sulfoximines, homocysteine sulfoximine, buthionine sulfones, penta-, hexa-, heptathionine sulfoximine),
- (metal) chelating agents (e.g. alpha-hydroxy fatty acids, palmitic acid, phytic acid, lactoferrin),
- alpha-hydroxy acids (for example citric acid, lactic acid, malic acid),
- humic acid, bile acid, bile extracts, bilirubin, biliverdin, boldin (= alkaloid from the plant Peumus boldus, boldo extract,
- EDTA, EGTA and derivatives thereof,
- unsaturated fatty acids and derivatives thereof (e.g. gamma-linolenic acid, linoleic acid, oleic acid),
- folic acid and derivatives thereof,
- ubiquinone and ubiquinol and derivatives thereof,
- vitamin C and derivatives (for example ascorbyl palmitate, Mg ascorbyl phosphate, ascorbyl acetate),
- tocopherols and derivatives (for example vitamin E acetate),
- vitamin A and derivatives (for example vitamin A palmitate),
- coniferyl benzoate of gum benzoin, rutic acid and derivatives thereof, alpha-glycosylrutin, ferulic acid, furfurylideneglucitol,
- butylhydroxytoluene (BHT), butylhydroxyanisole (BHA),
- nordihydroguaiacic acid, nordihydroguaiaretic acid, trihydroxybutyrophenone, uric acid and derivatives thereof, mannose and derivatives thereof,
- superoxide dismutase,
- zinc and derivatives thereof (for example ZnO, ZnSO4),
- selenium and derivatives thereof (for example selenomethionine),
- stilbenes and derivatives thereof (e.g. stilbene oxide, trans-stilbene oxide),
or mixtures of two or more of the aforementioned.

In a preferred embodiment, the antioxidant is selected from pentaerythrityl, tetra-di-t-butyl-hydroxyhydrocinnamate, nordihydroguaiaretic acid, ferulic acid, resveratrol, propyl gallate, butylhydroxytoluene (BHT), butylhydroxyanisole (BHA), ascorbyl palmitate, tocopherol, or mixtures of two or more of the aforementioned.

Preferably, the compositions according to the presently claimed invention comprise the antioxidant in a total amount of 0.001 to 25 wt.-%, preferably 0.005 to 10 wt.-%, more preferably 0.01 to 8 wt.-%, yet more preferably 0.025 to 7 wt.-%, even more preferably 0.05 to 5 wt.-%, based on the total weight of the composition.

In a preferred embodiment, the composition comprises Mixture I or Mixture II and at least one antioxidant and optionally at least one aroma chemical (X).

### Deodorant-active agents

Deodorizing compositions (deodorants and antiperspirants) counteract, mask or eliminate body odors. Body odors are formed through the action of skin bacteria on apocrine perspiration which results in the formation of unpleasant-smelling degradation products.

Preferably the deodorant-active agent is selected from anti-perspirants, esterase inhibitors, antibacterial agents, or mixtures of two or more of the aforementioned.

Suitable antiperspirants are selected from salts of aluminum, zirconium or zinc. Examples are aluminum chloride, aluminum chlorohydrate, aluminum dichlorohydrate, aluminum sesquichlorohydrate and complex compounds thereof, for example with 1,2-propylene glycol, aluminum hydroxyallantoinate, aluminum chloride tartrate, aluminum zirconium trichlorohydrate, aluminum zirconium tetrachlorohydrate, aluminum zirconium pentachlorohydrate and complex compounds thereof, for example with amino acids, such as glycine. Aluminum chlorohydrate, aluminum zirconium tetrachlorohydrate, aluminum zirconium pentachlorohydrate, or complex compounds thereof are preferably used.

Preferably, the anti-perspirant is selected from aluminum chloride, aluminum chlorohydrate, aluminum dichlorohydrate, aluminum sesquichlorohydrate, aluminum hydroxyallantoinate, aluminum chloride tartrate, aluminum zirconium trichlorohydrate, aluminum zirconium tetrachlorohydrate aluminum zirconium pentachlorohydrate, ormixtures of two or more of the aforementioned.

Where perspiration is present in the underarm region, extracellular enzymes-esterases, mainly proteases and/or lipases are formed by bacteria and split the esters present in the perspiration, releasing odors in the process. Suitable esterase inhibitors are for example trialkyl citrates, such as trimethyl citrate, tripropyl citrate, triisopropyl citrate, tributyl citrate and, in particular, triethyl citrate. Esterase inhibitors inhibit enzyme activity and thus reduce odor formation. The free acid is probably released by the cleavage of the citric acid ester and reduces the pH value of the skin to such an extent that the enzymes are inactivated by acylation. Other esterase inhibitors are sterol sulfates or phosphates such as, for example, lanosterol, cholesterol, campesterol, stigmasterol and sitosterol sulfate or phosphate, dicarboxylic acids and esters thereof, for example glutaric acid, glutaric acid monoethyl ester, glutaric acid diethyl ester, adipic acid, adipic acid monoethyl ester, adipic acid diethyl ester, malonic acid and malonic acid diethyl ester, hydroxycarboxylic acids and esters thereof, for example citric acid, malic acid, tartaric acid or tartaric acid diethyl ester, zinc glycinate and mixtures of two or more of the aforementioned.

Preferably, the esterase inhibitor is selected from trimethyl citrate, tripropyl citrate, triisopropyl citrate, tributyl citrate triethyl citrate, lanosterol, cholesterol, campesterol, stigmasterol, sitosterol sulfate, sitosterol phosphate, glutaric acid, glutaric acid monoethyl ester, glutaric acid diethyl ester, adipic acid, adipic acid monoethyl ester, adipic acid diethyl ester, malonic acid, malonic acid diethyl ester, citric acid, malic acid, tartaric acid, tartaric acid diethyl ester zinc glycinate, or mixtures of two or more of the aforementioned.

Preferably, the compositions according to the presently claimed invention comprise the esterase inhibitor in a total amount in the range of 0.01 to 20 wt.-%, preferably 0.1 to 10 wt.% and more particularly 0.5 to 5 wt.-%, based on the total weight of the composition.

The term "anti-bacterial agents" as used herein encompasses substances which have bactericidal and/or bacteriostatic properties. Typically these substances act against gram-positive bacteria such as, for example, 4-hydroxybenzoic acid and salts and esters thereof, N-(4-chlorophenyl)-N'-(3,4-dichlorophenyl)-urea, 2,4,4'-trichloro-2'-hydroxydiphenylether (triclosan), 4-chloro-3,5-dimethylphenol, 2,2'-methylene-bis-(6-bromo-4-chlorophenol), 3-methyl-4-(1-methylethyl)-phenol, 2-benzyl-4-chlorophenol, 3-(4-chlorophenoxy)-propane-1,2-diol, 3-iodo-2-propinyl butyl carbamate, chlorhexidine, 3,4,4'-trichlorocarbanilide (TTC), phenoxyethanol, glycerol monocaprate, glycerol monocaprylate, glycerol monolaurate (GML), diglycerol monocaprate (DMC), salicylic acid-N-alkylamides such as, for example, salicylic acid-n-octyl amide or salicylic acid-n-decyl amide.

Preferably, the antibacterial agent is selected from chitosan, phenoxyethanol, 5-chloro-2-(2,4-dichlorophenoxy)-phenol, 4-hydroxybenzoic acid and salts and esters thereof, N-(4-chlorophenyl)-N'-(3,4-dichlorophenyl)-urea, 2,4,4'-trichloro-2'-hydroxydiphenylether (triclosan), 4-chloro-3,5-dimethylphenol, 2,2'-methylene-bis-(6-bromo-4-chlorophenol), 3-methyl-4-(1-methylethyl)-phenol, 2-benzyl-4-chlorophenol, 3-(4-chlorophenoxy)-propane-1,2-diol, 3-iodo-2-propinyl butyl carbamate, chlorhexidine, 3,4,4'-trichlorocarbanilide (TTC), phenoxyethanol, glycerol monocaprate, glycerol monocaprylate, glycerol monolaurate (GML), diglycerol monocaprate (DMC), salicylic acid-N-alkylamides, or mixtures of two or more of the aforementioned.

Preferably, the composition according to the presently claimed invention comprises the antibacterial agent(s) in a total amount in the range of 0.01 to 5 wt.% and preferably 0.1 to 2 wt.-%, based on the total weight of the composition.

In a preferred embodiment, the composition comprises Mixture I or Mixture II and at least one deodorant active agent and optionally at least one aroma chemical (X).

### Surfactants

Preferably, the surfactant is selected from anionic, non-ionic, cationic, amphoteric, zwitterionic surfactant, or a mixture of two or more of the aforementioned. More preferably, the surfactant is an anionic surfactant.

Preferably, the compositions according to the invention contain the surfactant(s), in a total amount of 0 to 40 wt.%, more preferably 0 to 20 wt.%, more preferably 0.1 to 15 wt.%, and particularly 0.1 to 10 wt.%, based on the total weight of the composition.

Preferable nonionic surfactants are fatty alcohol polyglycol ethers, alkylphenol polyglycol ethers, fatty acid polyglycol esters, fatty acid amide polyglycol ethers, fatty amine polyglycol ethers, alkoxylated triglycerides, mixed ethers and mixed formals, optionally partly oxidized alk(en)yl oligoglycosides or glucuronic acid derivatives, fatty acid-N-alkyl glucamides, protein hydrolysates (particularly wheat-based vegetable products), polyol fatty acid esters, sugar esters, sorbitan esters, polysorbates and amine oxides. If the nonionic surfactants contain polyglycol ether chains, they may have a conventional homolog distribution, although they preferably have a narrow-range homolog distribution.

Zwitterionic surfactants are surface-active compounds which contain at least one quaternary ammonium group and at least one COO(-) or SO3(-) group in the molecule.

Particularly suitable zwitterionic surfactants are the so-called betaines, such as the N-alkyl-N,N-dimethyl ammonium glycinates, for example, cocoalkyl dimethyl ammonium glycinate, N-acylaminopropyl-N,N-dimethyl ammonium glycinates, for example, cocoacylaminopropyl dimethyl ammonium glycinate, and 2-alkyl-3-carboxymethyl-3-hydroxyethyl imidazolines, containing 8 to 18 carbon atoms in the alkyl or acyl group, and cocoacylaminoethyl hydroxyethyl carboxymethyl glycinate. The fatty acid amide derivative known under the CTFA name of Cocamidopropyl Betaine is particularly preferred.

Ampholytic surfactants are also suitable, particularly as co-surfactants. Ampholytic surfactants are surface-active compounds which, in addition to a C8 to C18 alkyl or acyl group, contain at least one free amino group and at least one -COOH or -SO3H group in the molecule and which are capable of forming inner salts. Examples of suitable ampholytic surfactants are N-alkyl glycines, N-alkyl propionic acids, N-alkylaminobutyric acids, N-alkyliminodipropionic acids, N-hydroxyethyl-N-alkylamidopropyl glycines, N-alkyl taurines, N-alkyl sarcosines, 2-alkylaminopropionic acids and alkylaminoacetic acids containing around 8 to 18 carbon atoms in the alkyl group. Particularly preferred ampholytic surfactants are N-cocoalkylaminopropionate, cocoacylaminoethyl aminopropionate and acyl sarcosine.

Anionic surfactants are characterized by a water-solubilizing anionic group such as, for example, a carboxylate, sulfate, sulfonate or phosphate group and a lipophilic group. Dermatologically safe anionic surfactants are known to the practitioner in large numbers from relevant textbooks and are commercially available. They are, in particular, alkyl sulfates in the form of their alkali metal, ammonium or alkanolammonium salts, alkylether sulfates, alkylether carboxylates, acyl isethionates, acyl sarcosinates, acyl taurines containing linear C12-C18 alkyl or acyl groups and sulfosuccinates and acyl glutamates in the form of their alkali metal or ammonium salts.

Particularly suitable cationic surfactants are quaternary ammonium compounds, preferably ammonium halides, more especially chlorides and bromides, such as alkyl trimethyl ammonium chlorides, dialkyl dimethyl ammonium chlorides and trialkyl methyl ammonium chlorides, for example, cetyl trimethyl ammonium chloride, stearyl trim ethyl ammonium chloride, distearyl dimethyl ammonium chloride, lauryl dimethyl ammonium chloride, lauryl dimethyl benzyl ammonium chloride and tricetyl methyl ammonium chloride. In addition, the readily biodegradable quaternary ester compounds, such as, for example, the dialkyl ammonium methosulfates and methyl hydroxyalkyl dialkoyloxyalkyl ammonium methosulfates marketed under the name of Stepantexe and the corresponding products of the Dehyquart^{®} series, may be used as cationic surfactants. "Esterquats" are generally understood to be quaternized fatty acid triethanolamine ester salts. They can provide the compositions with particular softness. They are known substances which are prepared by the relevant methods of organic chemistry. Other cationic surfactants suitable for use in accordance with the invention are the quaternized protein hydrolysates.

Due to the characteristic sensory property of compound of formula (I) and its substantivity, tenacity as well as stability, it can especially be used to provide an odor, preferably a fragrance impression to surfactant-containing compositions such as, for example, cleaners (in particular laundry care products and all-purpose cleaners). It can preferably be used to impart a long-lasting notes selected from woody note, pine like note, amber note, fresh note, musky note, fruity note, green note, Floral note, powdery note, spicy note to a surfactant comprising composition.

In a preferred embodiment, the composition comprises Mixture I or Mixture II and at least one surfactant and optionally at least one aroma chemical (X).

Suitable compositions are for example perfume compositions, body care compositions (including cosmetic compositions and products for oral and dental hygiene), hygiene articles, cleaning compositions (including dishwashing compositions), textile detergent compositions, compositions for scent dispensers, foods, food supplements, pharmaceutical compositions, and crop protection compositions.

Perfume compositions can be selected from fine fragrances, air fresheners in liquid form, gel-like form or a form applied to a solid carrier, aerosol sprays, scented cleaners, perfume candles, or oils, such as lamp oils or oils for massage.

Examples for fine fragrances are perfume extracts, Eau de Parfums, Eau de Toilettes, Eau de Colognes, Eau de Solide and Extrait Parfum.

Body care compositions include cosmetic compositions and products for oral and dental hygiene, and can be selected from after-shaves, pre-shave products, splash colognes, solid and liquid soaps, shower gels, shampoos, shaving soaps, shaving foams, bath oils, cosmetic emulsions of the oil-in-water type, of the water-in-oil type and of the water-in-oil-in-water type, such as e.g. skin creams and lotions, face creams and lotions, sunscreen creams and lotions, after-sun creams and lotions, hand creams and lotions, foot creams and lotions, hair removal creams and lotions, after-shave creams and lotions, tanning creams and lotions, hair care products such as e.g. hairsprays, hair gels, setting hair lotions, hair conditioners, hair shampoo, permanent and semi-permanent hair colorants, hair shaping compositions such as cold waves and hair smoothing compositions, hair tonics, hair creams and hair lotions, deodorants and antiperspirants such as e.g. underarm sprays, roll-ons, deodorant sticks and deodorant creams, products of decorative cosmetics such as e.g. eye-liners, eye-shadows, nail varnishes, make-ups, lipsticks and mascara, and products for oral and dental hygiene, such as toothpaste, dental floss, mouth wash, breath fresheners, dental foam, dental gels and dental strips.

Hygiene articles can be selected from joss sticks, insecticides, repellents, propellants, rust removers, perfumed freshening wipes, armpit pads, baby diapers, sanitary towels, toilet paper, cosmetic wipes, pocket tissues, dishwasher, and deodorizer.

Cleaning compositions, such as e.g. cleaners for solid surfaces, can be selected from perfumed acidic, alkaline and neutral cleaners, such as e.g. floor cleaners, window cleaners, dishwashing compositions both for handwashing and machine washing use, bath and sanitary cleaners, scouring milk, solid and liquid toilet cleaners, powder and foam carpet cleaners, waxes and polishes such as furniture polishes, floor waxes, shoe creams, disinfectants, surface disinfectants and sanitary cleaners, brake cleaners, pipe cleaners, limescale removers, grill and oven cleaners, algae and moss removers, mold removers, facade cleaners.

Textile detergent compositions can be selected from liquid detergents, powder detergents, laundry pre-treatments such as bleaches, soaking agents and stain removers, fabric softeners, washing soaps, washing tablets.

Food means a raw, cooked, or processed edible substance, ice, beverage, or ingredient used or intended for use in whole or in part for human consumption, or chewing gum, gummies, jellies, and confectionaries.

A food supplement is a product intended for ingestion that contains a dietary ingredient intended to add further nutritional value to the diet. A dietary ingredient may be one, or any combination, of the following substances: a vitamin, a mineral, an herb or other botanical, an amino acid, a dietary substance for use by people to supplement the diet by increasing the total dietary intake, a concentrate, metabolite, constituent, or extract. Food supplements may be found in many forms such as tablets, capsules, soft gels, gel caps, liquids, or powders.

Pharmaceutical compositions comprise compositions which are intended for use in the diagnosis, cure, mitigation, treatment, or prevention of disease as well as articles (other than food) intended to affect the structure or any function of the body of man or other animals.

Crop protection compositions comprise compositions which are intended for the managing of plant diseases, weeds, and other pests (both vertebrate and invertebrate) that damage agricultural crops and forestry.

Preferably, the compositions according to the invention further comprises at least one auxiliary agent selected from the group consisting of preservatives, abrasives, anti-acne agents, agents to combat skin aging, anti-cellulite agents, antidandruff agents, antiinflammatory agents, irritation-preventing agents, irritation-alleviating agents, astringents, sweat-inhibiting agents, antiseptics, anti-statics, binders, buffers, carrier materials, chelating agents, cell stimulants, care agents, hair removal agents, emulsifiers, enzymes, essential oils, fibers, film formers, fixatives, foam formers, foam stabilizers, substances for preventing foaming, foam boosters, fungicides, gelling agents, gel-forming agents, hair care agents, hair shaping agents, hair smoothing agents, moisture-donating agents, moisturizing substances, humectant substances, bleaching agents, strengthening agents, stain removal agents, optical brighteners, impregnating agents, soil repellents, friction-reducing agents, lubricants, moisturizing creams, ointments, opacifiers, plasticizers, covering agents, polish, shine agents, polymers, powders, proteins, refatting agents, exfoliating agents, silicones, skin-calming agents, skin-cleansing agents, skin care agents, skin-healing agents, skin lightening agents, skin-protective agents, skin-softening agents, cooling agents, skin-cooling agents, warming agents, skin-warming agents, stabilizers, UV-absorbent agents, UV filters, fabric softeners, suspending agents, skin-tanning agents, thickeners, vitamins, waxes, fats, phospholipids, saturated fatty acids, mono or polyunsaturated fatty acids, alpha hydroxy acids, polyhydroxy fatty acids, liquefiers, dyes, color-protection agents, pigments, anti-corrosives, polyols, electrolytes and silicone derivatives.

Preparation of compositions and methods to impart an aroma impression to a composition:
One embodiment of the present invention is directed to a method for preparing a composition comprising Mixture I or Mixture II and
   (i) at least one aroma chemical (X) other than compounds according to the present invention specifically, compounds of formula (I), compounds of formula (II), and compounds of formula (III), including the stereoisomers thereof, or
   (ii) at least one non-aroma chemical carrier, or
   both of (i) and (ii).
For example, the method can be carried out by mixing the Mixture I or Mixture II and:
   (i) at least one aroma compound(X) other than compounds according to the present invention, specifically, compounds of formula (I), compounds of formula (II), and compounds of formula (III), including the stereoisomers thereof, or
   (ii) at least one non-aroma chemical carrier, or
   (iii) both of (i) and (ii).

The invention is also directed to a method for boosting the aroma impression of a composition, wherein the method comprises incorporating Mixture I or Mixture II into a composition.

In particular, the invention is directed to a method of preparing a perfume composition, body care composition, hygiene article, cleaning composition, textile detergent composition, composition for scent dispensers, food, food supplement, pharmaceutical composition or crop protection composition, comprising including compound of formula (I) into a perfume composition, body care composition, hygiene article, cleaning composition, textile detergent composition, composition for scent dispensers, food, food supplement, pharmaceutical composition, or crop protection composition.

In one embodiment the invention is directed to a method for imparting a note reminiscent of woody note, pine like note, amber note, fresh note, musky note, fruity note, green note, Floral note, powdery note, spicy note ,elements to a perfume composition, body care composition, hygiene article, cleaning composition, textile detergent composition, composition for scent dispensers, food, food supplement, pharmaceutical composition or crop protection composition, which comprises including compound of formula (I) into a perfume composition, body care composition, hygiene article, cleaning composition, textile detergent composition, composition for scent dispensers, food, food supplement, pharmaceutical composition, or crop protection composition.

### Amounts

Generally, the total amount of compounds of compound of formula (I), compound of formula (II) and compound of formula (III) in the compositions, methods and uses according to the present invention is typically adapted to the particular intended use or the intended application and can, thus, vary over a wide range. As a rule, the customary standard commercial amounts for aroma chemicals, preferably for scents are used.

Preferably the compositions according to the invention comprise compounds of formula (I) in a total amount of 0.001 to 99.9 wt.%, based on the total weight of the composition.

Particularly, the compositions comprise compound of formula (I), compound of formula (II) and optionally compound of formula (III) in a total amount of 0.001 to 99.5 wt.%, preferably of 50 to 99 wt.%, more preferably of 80 to 95 wt.% and in particular of 90 to 95 wt.%, based on the total weight of the composition.

Particularly, the compositions comprise compounds of compound of formula (I), compound of formula (II) and optionally compound of formula (III) in a total amount of 0.005 to 80 wt.%, preferably of 0.1 to 30 wt.%, more preferably of 1 to 20 wt.%, and in particular of 5 to 15 wt.%, based on the total weight of the composition.

Particularly, the compositions comprise the compounds of compound of formula (I), compound of formula (II) and optionally compound of formula (III) in a total amount of 0.001 to 20 wt.%, preferably of 0.005 to 6 wt.%, more preferably of 0.05 to 4 wt.%, and in particular of 0.1 to 3 wt.%, based on the total weight of the composition.

### Process:

In an embodiment the present invention relates to a process for preparing the mixture of compound of formula (I) and compound of formula (II), comprising at least the step of
a) Hydroformylation of the compound of formula (IV) 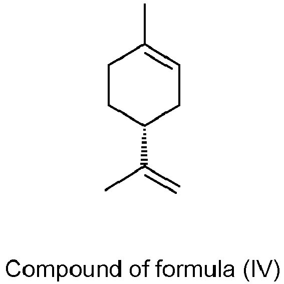 in the presence of a metal catalyst to produce the mixture of compound of formula (I) and compound of formula (II), and
b) optionally, purifying the mixture of compound of formula (I) and compound of formula (II).

### Embodiments

In the following, there is provided a list of embodiments to further illustrate the present disclosure without intending to limit the disclosure to the specific embodiments listed below
1. Use of a mixture comprising compound of formula (I) 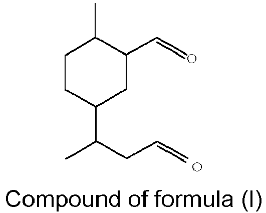
   or its salts or stereoisomer thereof
   and
   compound of formula (II) 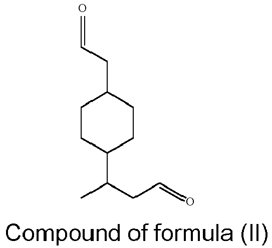
   or its salts or stereoisomer thereof
   to impart an aroma impression to a composition.
2. The use of the mixture according to embodiment 1, wherein the weight ratio of compound of formula (I) and (II) is in the range 1:99 to 99:1.
3. The use of the mixture according to embodiment 1, wherein the weight ratio of the of compound of formula (I) and (II) is in the range 10:90 to 90:10.
4. The use of the mixture according to embodiment 1, wherein the weight ratio of the of compound of formula (I) and (II) is in the range 20:80 to 80:20.
5. The use of the mixture according to embodiment 1, wherein the weight ratio of the of compound of formula (I) and (II) is in the range 25 :75 to 75:25.
6. The use of the mixture according to embodiment 1, wherein the mixture further comprises a compound of formula (III) 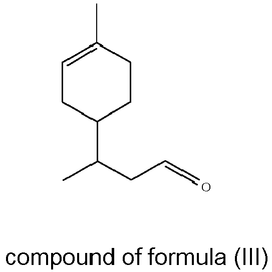 to impart an aroma impression to a composition.
7. The use of the mixture according embodiment 6, wherein the weight ratio of the mixture of compound of formula (I), compound of formula (II) and compound of formula (III) is in the range of 0.05-80:0.05-15:0.1-99.9 respectively.
8. A method of imparting an aroma impression to a composition comprising at least the step of adding the mixture according to any of the embodiments 1 to 5 or any of the embodiments 6 to 7 to a composition.
9. The use or method according to any of the embodiments 1 to 8, wherein the composition is selected from the group consisting of perfume compositions, body care compositions, hygiene articles, cleaning compositions, textile detergent compositions, compositions for scent dispensers, foods, food supplements, pharmaceutical compositions and crop protection compositions.
10. The use or method according to any of the embodiments 1 to 8, wherein the aroma impression is selected from the group of Agrumic note, Chrysanthemum note, Floral note, Muguet note, Watery note, Waxy note, Citric note, Lemon note, Aldehydic note, Green note or a combination of two or more notes.
11. The use or method according to any of the embodiments 1 to 8, wherein the mixture of compounds of formula (I) and (II) is used in a total amount in the range of ≥ 0.001 wt.% to ≤ 70.0 wt.%, based on the total weight of the composition.
12. A composition comprising,
   (i) the mixture according to any of the embodiments 1 to 5 or a mixture according to any of embodiments 6 to 7 and,
   (ii) at least one aroma chemical other than compounds of formula (I),(II) and (III) or
   (iii) at least one non-aroma chemical carrier, or
   (iv) both of (ii) and (iii).
13. The composition according to embodiment 12, wherein the non-aroma chemical carrier (iii) is selected from the group consisting of surfactants, oil components, antioxidants, deodorant-active agents and solvents.
14. The composition according to any of the embodiments 12 to 13, wherein the composition is selected from the group consisting of perfume compositions, body care compositions, hygiene articles, cleaning compositions, textile detergent compositions, compositions for scent dispensers, foods, food supplements, pharmaceutical compositions and crop protection compositions.
15. A process for preparing the mixture of compound of formula (I) and compound of formula (II) according to any of the embodiments 1 to 5, comprising at least the step of
   a) Hydroformylation of the compound of formula (IV) 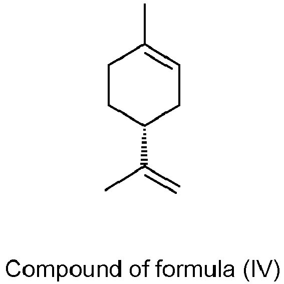 in the presence of a metal catalyst to produce the mixture of compound of formula (I) and compound of formula (II), and
   b) optionally, purifying the mixture of compound of formula (I) and compound of formula (II).

### Examples:

The present invention is illustrated in detail by non-restrictive working examples which follow. More particularly, the test methods specified hereinafter are part of the general disclosure of the application and are not restricted to the specific working examples.

### Analytical method:

¹³C NMR spectra were recorded on a Bruker AV3 500 MHz spectrometer and referenced to the residual carbon resonance peak of the solvent CDCl₃.

### GC analysis:

GC analyses were performed using an Agilent 7890 B equipped with an Optima 1 column (30m, 0.32 mm, 0.5µm).

| | |
|---|---|
| Temperature program: | 50 °C, 5 min isotherm |
| | 20 K/min to 300°C, 22.5 min isotherm |
| Constant Flow: | 1.6005 ml/min |
| Carrier gas: | Helium |
| Injector temperature: | 280°C |
| Detector temperature: | 320°C |

### Example 1:

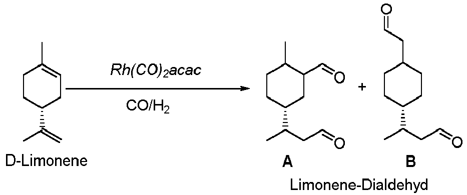

A 300 ml PREMEX HC autoclave was charged with the 30 mg Dicarbonyl(acetylacetonato) rhodium(I) (Rh(CO)2acac ) dissolved in 120 g Limonene. The autoclave was stirred with 700 U/min and pressurized with 580 bar oxo gas (CO:H₂ = 1:1). After depressurizing to ambient pressure the stirring was increased to 1000 U/min and the autoclave was pressurized with 50 bar oxo gas (CO:H2 = 1:1). The autoclave was heated to 120°C internal temperature before the oxo gas pressure was increased to 580 bar. After the reaction mixture was stirred for 12h at the same conditions, the autoclave was cooled down to room temperature before depressurizing to ambient pressure. The autoclave was pressurized with 20 bar N₂ and depressurized to ambient pressure for three times before the crude limonen-dialdehyd was analyzed via gas chromatography.

Conversion: 98.9%, Yield: 85.4%, Selectivity: 86.3%

The crude Limonen-Dialdehyde was further purified via vacuum distillation using a 20 cm packed fractionating column with 3x3 mm Raschig rings. A boiling capillary was used to ensure an inert argon atmosphere in the distillation sump. 42.2 g of Limonen-dialdehyde were obtained at <1 mbar and 103.3 - 107.6°C head temperature with 97.4% purity determined by gas chromatography. The isomeric ratio A : B = 80 : 10 was determined by ¹³C NMR.

### 3. Olfactory evaluation

In order to test the quality and intensity of the compounds of the present invention, scent strip tests were performed.

For this purpose, strips of absorbent paper were dipped into a solution containing 1 to 10 wt.% of the compound to be tested in triethyl citrate. After evaporation of the solvent (about 30 s) the scent impression was olfactively evaluated by a trained perfumer.

Aroma impression of Mixtures of the present invention are indicated in the below table I.
Mixture (I)- Compound of formula (I) + Compound of formula (II)
Mixture (II)- Compound of formula (I) + Compound of formula (II)+ Compound of formula (III)

### Structures of the compound

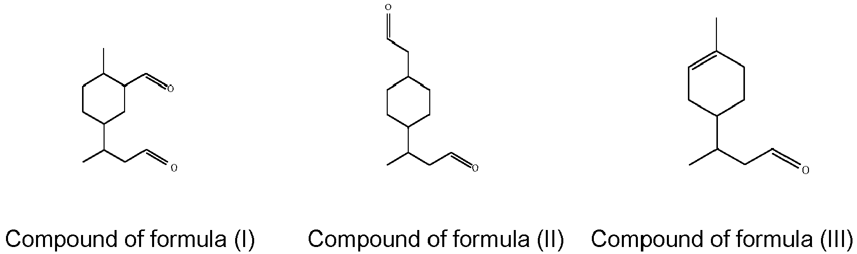

**Table I:**

| Mixture / compound | Ratio | Olfactory Impression |
|---|---|---|
| Mixture (I) | 80:10 (I: II) | Agrumic, Chrysanthemum, Floral, Muguet, Watery, Waxy |
| Mixture (II) | 79.2:9.9:1 (I: II: III) | Agrumic, Chrysanthemum, Floral, Muguet, Watery, Waxy with a very minor citric note |
| Mixture (II) | 72:9 :10 (I: II: III) | Agrumic, Chrysanthemum, Floral, Muguet, Watery, Waxy with minor citric note |
| Mixture (II) | 40:5:50 (I: II: III) | Agrumic, Chrysanthemum, Floral, Muguet, Watery, Waxy and citric. The floral aspects are clearly dominant vs the citric note. |
| Mixture (II) | 8:1:90 (I: II: III) | Citrus, lemon, aldehydic, green but with some floral aspects |
| Mixture (II) | 0.8:0.1 :99 (I: II: III) | Citrus, lemon, aldehydic, green but with some floral nuancen much less citric than pure III (mono) |
| "Compound of formula (III) | 100 | Citrus, lemon, green, aldehydic, herbal |

| | | |
|---|---|---|
| # not as per the invention | | |

### Advantageous compositions

The mixtures indicated in table I were formulated as compositions according to tables 2 and 3. Mixtures from table I are labelled as "Compound P" in table 2 and 3.

**Table II : Compositions 1A and 1B**

| | 1A | 1B |
|---|---|---|
| Lactone C10 gamma (5-hexyloxolan-2-one) | 2 | 2 |
| Bourgeonal (3-(4-tert-butylphenyl)propanal) | 2 | 2 |
| Citronellol | 3 | 3 |
| Aldehyde C-14 (5-heptyloxolan-2-one) | 3 | 3 |
| Allyl heptylate | 4 | 4 |
| Amber core (1-(2-tert-butylcyclohexyl)oxybutan-2-ol) | 4 | 4 |
| Ethyl-2-methyl butyrate | 4 | 4 |
| Geranyl acetate | 5 | 5 |
| Helional (3-(1,3-benzodioxol-5-yl)-2-methylpropanal) | 10 | 10 |
| Manzanate (ethyl 2-methylpentanoate) | 10 | 10 |
| Amberwood (ethoxymethoxycyclododecane) | 10 | 10 |
| Hexyl acetate | 11 | 11 |
| Benzyl salicylate | 12 | 12 |
| Magnolan (2,4-dimethyl-4,4a,5,9b-tetrahydroindeno[1,2-d][1,3]dioxine) | 15 | 15 |
| Verdox (2-tert-butylcyclohexyl) acetate) | 25 | 25 |
| Bergamot oil bergaptene free | 25 | 25 |
| Linalol | 30 | 30 |
| Dipropylene glycol | 45 | 45 |
| Iso E Super (Tetramethyl acetyloctahydronaphthalenes) | 110 | 110 |
| Pyranol (4-methyl-2-(2-methyl propyl)oxan-4-ol) | 170 | 170 |
| Hedione (methyl 3-oxo-2-pentylcyclopentaneacetate) | 200 | 200 |
| Galaxolide 50% IPM (1,3,4,6,7,8-hexahydro-4,6,6,7,8,8-hexamethylcyclopenta(g)-2-benzopyran 50% in isopropyl myristate) | 300 | 300 |
| compound P | 20 | 50 |
| Total ( part by weight) | 1020 | 1050 |

**Table III: Compositions 2A and 2B**

| | 2A | 2B |
|---|---|---|
| Raspberry ketone (4-(4-hydroxyphenyl)butan-2-one) | 4 | 4 |
| Vanitrope (2-ethoxy-5-prop-1-enylphenol) | 6 | 6 |
| Cyclamen aldehyde (at least 90% 2-methyl-3-(p-isopropylphenyl)-propionaldehyde; secondary component: 5% 3-(p-cumenyl)-2-methylpropionic acid) | 10 | 10 |
| Bicyclononalactone (3,4,4a,5,6,7,8,8a-octahydrochromen-2-one) | 10 | 10 |
| Aldehyde C-14 (5-heptyloxolan-2-one) | 14 | 14 |
| Ethylvanillin (3-ethoxy-4-hydroxybenzaldehyde) | 16 | 16 |
| Heliotropine (1,3-benzodioxole-5-carbaldehyde) | 20 | 20 |
| Iso E Super (tetramethyl acetyloctahydronaphthalenes) | 20 | 20 |
| Sandela (3-[5,5,6-trimethylbicyclo[2.2.1]hept-2-yl]cyclohexan-1-ol) | 30 | 30 |
| Vanillin isobutyrate ((4-formyl-2-methoxyphenyl) 2-methylpropanoate) | 40 | 40 |
| Aldehyde C-18 (5-pentyloxolan-2-one) | 50 | 50 |
| Benzyl salicylate | 60 | 60 |
| Hexyl cinnamic aldehyde (2-(phenylmethylidene)octanal) | 70 | 70 |
| Hedione (methyl 3-oxo-2-pentylcyclopentaneacetate) | 130 | 130 |
| Pyranol (4-methyl-2-(2-methyl propyl)oxan-4-ol) | 150 | 150 |
| Ethylene brassylate (1,4-dioxacycloheptadecane-5,17-dione) | 170 | 170 |
| Galaxolide 50% IPM (1,3,4,6,7,8-hexahydro-4,6,6,7,8,8-hexamethylcyclopenta(g)-2-benzopyran 50% in isopropyl myristate) | 200 | 200 |
| compound P | 20 | 50 |
| Total ( part by weight) | 1020 | 1050 |

Composition according to table II and table III namely 1A, 1B, 2A,2B could be included in various compositions selected from, Deo pump spray, Clean hair-conditioner, Face wash gel, Foam bath concentrate, Hair gel, Self-foaming bodywash, Sprayable sun care emulsion, Sprayable sun protection emulsion, Emollient facial gel, 2-phases oil foam bath, Shampoos, Shower bath, Hydro-alcoholic AP/Deo pump spray, Aerosol, Aqueous/alcoholic AP/Deo roll-on, Styling Gel Type "Out of Bed", Shaving Foam, Sensitive skin Baby shampoo, Body wash for Sensitive Skin, Gloss Enhancing Shampoo for Sensitive Scalp, Deo Stick, Baby Wipe, After shave balm, Face Gel, Face Day Care Cream, Face Cleanser, Body lotion, Sun Care SPF50+, Sprayable Lotion, Hand dish cleaner - regular, Hand dish cleaner - concentrate, Sanitary cleaner - concentrate, All-purpose cleaner, Anti-bacterial fabric softener, Detergent composition, Powder detergent composition and Liquid detergent composition.

A person skilled in art may be well versed with the various formulations for the above-mentioned products.

Compositions 1A, 1B, 2A and 2B can for example be formulated in specific formulations as disclosed in IP.com Number: IPCOM000258614D entitled New Aroma Chemicals pages 6 to 46, Table 1 to Table D13, wherein the "Fragrance Composition 1A" is replaced by identical amounts of compositions 1A, 1B, 2A or 2B.

## Claims

1. Use of a mixture comprising compound of formula (I) 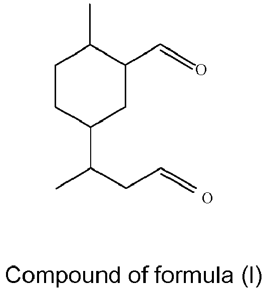
or its salts or stereoisomer thereof
and
compound of formula (II) 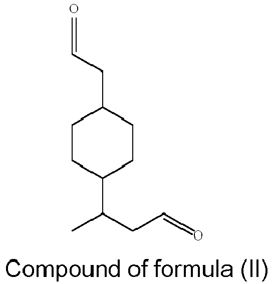
or its salts or stereoisomer thereof
to impart an aroma impression to a composition.

2. The use of the mixture according to claim 1, wherein the weight ratio of compound of formula (I) and (II) is in the range 1:99 to 99:1.

3. The use of the mixture according to claim 1, wherein the weight ratio of the of compound of formula (I) and (II) is in the range 10:90 to 90:10.

4. The use of the mixture according to claim 1, wherein the weight ratio of the of compound of formula (I) and (II) is in the range 20:80 to 80:20.

5. The use of the mixture according to claim 1, wherein the weight ratio of the of compound of formula (I) and (II) is in the range 25 :75 to 75:25.

6. The use of the mixture according to claim 1, wherein the mixture further comprises a compound of formula (III) 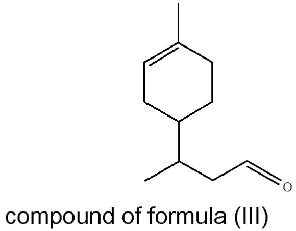 to impart an aroma impression to a composition.

7. The use of the mixture according to claim 6, wherein the weight ratio of the mixture of compound of formula (I), compound of formula (II) and compound of formula (III) is in the range of 0.05-80:0.05-15:0.1-99.9 respectively.

8. A method of imparting an aroma impression to a composition comprising at least the step of adding the mixture according to any of the claim 1 to 5 or any of the claims 6 to 7 to a composition.

9. The use or method according to any of the claims 1 to 8, wherein the composition is selected from the group consisting of perfume compositions, body care compositions, hygiene articles, cleaning compositions, textile detergent compositions, compositions for scent dispensers, foods, food supplements, pharmaceutical compositions, and crop protection compositions.

10. The use or method according to any of the claims 1 to 8, wherein the aroma impression is selected from the group of Agrumic note, Chrysanthemum note, Floral note, Muguet note, Watery note, Waxy note, Citric note, Lemon note, Aldehydic note, Green note or a combination of two or more notes.

11. The use or method according to any of the claims 1 to 8, wherein the mixture of compounds of formula (I) and (II) is used in a total amount in the range of ≥ 0.001 wt.% to ≤ 70.0 wt.%, based on the total weight of the composition.

12. A composition comprising,
(i) the mixture according to any of the claims 1 to 5 or a mixture according to any of claims 6 to 7 and,
(ii) at least one aroma chemical other than compounds of formula (I) ,(II) and (III) or
(iii) at least one non-aroma chemical carrier, or
(iv) both of (ii) and (iii).

13. The composition according to claim 12, wherein the non-aroma chemical carrier (iii) is selected from the group consisting of surfactants, oil components, antioxidants, deodorant-active agents and solvents.

14. The composition according to any of the claims 12 to 13, wherein the composition is selected from the group consisting of perfume compositions, body care compositions, hygiene articles, cleaning compositions, textile detergent compositions, compositions for scent dispensers, foods, food supplements, pharmaceutical compositions and crop protection compositions.

15. A process for preparing the mixture of compound of formula (I) and compound of formula (II) according to any of the claims 1 to 5, comprising at least the step of
a) Hydroformylation of the compound of formula (IV) 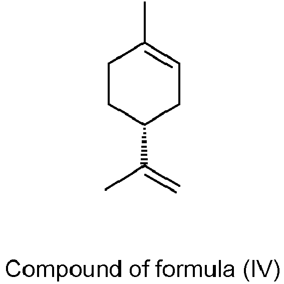 in the presence of a metal catalyst to produce the mixture of compound of formula (I) and compound of formula (II), and
b) optionally, purifying the mixture of compound of formula (I) and compound of formula (II).
